# EUROPÄISCHE PATENTSCHRIFT

(11) **EP 3 821 987 B1**
(45) Veröffentlichungstag und Bekanntmachung des Hinweises auf die Patenterteilung: **28.09.2022**
(21) Anmeldenummer: 19209529.7
(22) Anmeldetag: 15.11.2019
(51) Int. Cl.: B05B 11/00

(54) **FLÜSSIGKEITSSPENDER MIT FLASCHENBELÜFTUNG**
FLUID DISPENSER WITH BOTTLE VENTILATION
DISTRIBUTEUR DE LIQUIDE À AÉRATION DE BOUTEILLE

(43) Veröffentlichungstag der Anmeldung: 19.05.2021
(73) Patentinhaber: Aptar Radolfzell GmbH, 78315 Radolfzell (DE)
(72) Erfinder: Heinzle, Christian, 78224 Singen (DE)
(74) Vertreter: Witte, Weller und Partner Patentanwälte mbB Stuttgart

(56) Entgegenhaltungen:
- EP-A1- 2 130 610
- WO-A2-2007/096049
- DE-T2- 69 500 443

## Beschreibung

Die Erfindung betrifft einen Flüssigkeitsspender, insbesondere zum Austrag pharmazeutischer oder kosmetischer Flüssigkeiten. Ein solcher Flüssigkeitsspender kann beispielsweise zum Austrag der Flüssigkeit in Form von Tropfen, in Form eines Sprühnebels oder in Form eines Jets ausgebildet sein.

Ein erfindungsgemäßer Flüssigkeitsspender verfügt wie ein gattungsgemäßer Flüssigkeitsspender über einen Flüssigkeitsspeicher zur Lagerung von Flüssigkeit vor dem Austrag sowie über eine am Flüssigkeitsspeicher angebrachte Austragvorrichtung mit einer Austragöffnung zum Austrag der Flüssigkeit. Weiterhin verfügt er über eine Pumpeinrichtung zur manuellen Betätigung mit einer volumetrisch veränderlichen Pumpkammer, die an einer Eingangsseite kommunizierend mit dem Flüssigkeitsspeicher und an einer Ausgangsseite kommunizierend mit der Austragöffnung verbunden ist, so dass Flüssigkeit aus dem Flüssigkeitsspeicher in eine umgebende Atmosphäre oder auf eine Oberfläche abgegeben werden kann.

Durch die Abgabe von Flüssigkeit entleert sich der Flüssigkeitsspeicher nach und nach. Um im Flüssigkeitsspeicher einen dadurch erzeugten Unterdruck wieder abzubauen oder zumindest zu verringern, verfügen gattungsgemäße und erfindungsgemäße Flüssigkeitsspender über eine Austragvorrichtung, die ihrerseits über einen Belüftungskanal verfügen, mittels dessen ein einfließenden Luftstrom zum Druckausgleich zwischen dem Flüssigkeitsspeicher und einer umgebenden Atmosphäre ermöglicht wird. Dabei sind sowohl Gestaltungen bekannt, bei denen einströmende Luft in einen durch eine verformbare Beutelwandung von der Flüssigkeit getrennten Umgebungsbereich gelangt, als auch solche, bei denen die einströmende Luft unmittelbar in den Bereich der im Flüssigkeitsspeicher gelagerten Flüssigkeit gelangt.

Der Belüftungskanal stellt insbesondere bei Gestaltungen, bei denen die Luft unmittelbar in den Bereich der Flüssigkeit gelangt, ein mögliche Störungsquelle dar, da es passieren kann, dass die Flüssigkeit durch den Belüftungskanal hindurch ausströmt, insbesondere wenn der Flüssigkeitsspender sich in einer Überkopflage befindet, beispielsweise in Reisegepäck.

Aus Dokument DE 69500443 T2 ist eine Vordruckpumpe für die Zerstäubung einer Flüssigkeit aus einem Vorratsbehälter unter Druck bekannt. Diese verfügt über einen Vordruckkolben in einer Vordruckkammer sowie in einerWand im Bereich des Vordruckkolbens über eine Luftausgleichungsöffnung. Die Luftausgleichungsöffnung wird beim Betätigen der Vordruckpumpe durch eine Lippe des Vordruckkolbens freigegeben, so dass Luft durch die Luftausgleichungsöffnung einströmen kann und durch einen Kapillarkanal in den Vorratsbehälter gelangen kann.

Aus Dokument WO 2007/096049 A2 ist eine Dosiervorrichtung zur Abgabe einer Flüssigkeit bekannt. Zur Ermöglichung eines Druckausgleichs in einem Mediumspeicher der Dosiervorrichtung sind ein Kapillarkanal sowie eine Filteranordnung vorgesehen.

### AUFGABE UND LÖSUNG

Aufgabe der Erfindung ist es, einen gattungsgemäßen Flüssigkeitsspender zur Verfügung zu stellen, der die Gefahr eines Auslaufens vermindert.

Hierzu wird erfindungsgemäß ein Flüssigkeitsspender gemäß den Ansprüchen vorgeschlagen.

Das Belüftungsventil verhindert im geschlossenen Zustand das Einströmen von Luft und damit auch das Austreten von Flüssigkeit, die in ungewünschter Weise in den Belüftungskanal eingedrungen ist. Um das Belüftungsventil baulich einfach zu gestalten, wird es vorzugsweise durch einander gegenüberliegende Ventilflächen insbesondere an starren Gehäusebauteilen gebildet, die in einer Endlage aneinander anliegen und damit das Belüftungsventil schließen. Vorzugsweise weist die Austragvorrichtung eine Rückstellfeder auf, die insbesondere zur Rückstellung der Pumpeinrichtung in einer Ausgangslage dient. Diese Rückstellfeder dient vorzugsweise zusätzlich gleichzeitig als Ventilfeder, deren Federkraft die Ventilflächen aneinander drückt. Die Ventilflächen des Belüftungsventils liegen vorzugsweise mit einem mittleren Anpressdruck von 0,5 N/mm² bis 1,5 N/mm² aneinander an. Die Auslegung der Feder erfolgt vorzugsweise primär in Hinblick auf die gewünschte Betätigungskraft. Es ist daher nur in eingeschränkter Weise möglich, die Dichtwirkung des Belüftungsventils zu steigern. Ein wirtschaftlich sinnvolles Belüftungsventil ist daher gegebenenfalls so ausgelegt, dass nicht vollständig gewährleistet ist, dass die Verschlusswirkung des Ventils auch nach längerer Nutzung noch perfekt gegeben ist.

Um dennoch auch bei jenen Flüssigkeitsspendern, die bereits im Lieferzustand ein nicht perfekt dichtendes Belüftungsventil aufweisen oder bei denen nach längerer Nutzungszeit oder Liegezeit die Verschlusswirkung des Ventils nachlässt, ein Auslaufen zu verhindern, ist zusätzlich zu dem Ventil der genannte Kapillarkanalabschnitt vorgesehen, der auch für den Fall eines nur noch begrenzt wirksamen Belüftungsventils üblicherweise das Auslaufen von Flüssigkeit verhindern oder zumindest verzögern kann.

Der Kapillarkanalabschnitt ist ein Teil des Belüftungskanals. Durch seine vergleichsweise große Länge und/oder durch einen geringeren Querschnitt bzw. durch den sich daraus ergebenden hohen Strömungswiderstand verzögert er das ungewünschte Ausströmen von Flüssigkeit deutlich. Der Kapillarkanalabschnitt weist eine minimale lichte Querschnittsfläche auf, die an der engsten Stelle des Belüftungskanals maximal 0,1 mm beträgt. Insbesondere vorzugsweise beträgt der Querschnitt des Kapillarkanalabschnitts mindestens über eine Länge von 10 mm, vorzugsweise mindestens über eine Länge von 30 mm und insbesondere vorzugsweise mindestens über eine Länger von 50 mm, maximal 0,1 mm.

Die mittlere Querschnittsfläche des Kapillarkanalabschnitts, insbesondere bezogen auf einen Teilabschnitt von mindestens 10 mm Länge, beträgt vorzugsweise weniger als 0,05 mm², vorzugsweise weniger als 0,02 mm², insbesondere vorzugsweise weniger als 0,01 mm².

Vorzugsweise weist der Kapillarkanalabschnitt mindestens eine Stelle auf, an der der lichte Querschnitt kleiner ist als 0,02 mm². Dies verursachte eine signifikante Verzögerung beim ungewollten Austritt von Flüssigkeit durch den Belüftungskanal.

Der Kapillarkanalabschnitt weist vorzugsweise, insbesondere bezogen auf einen Teilabschnitt von mindestens 10 mm Länge, einen Quotienten aus der Länge des Kapillarkanalabschnitts bzw. dieses Teilabschnitts geteilt durch seine mittlere Querschnittsfläche auf, der größer als 300 mm⁻¹ ist, insbesondere größer als 1000 mm⁻¹.

Bevorzugt ist es, wenn das Belüftungsventil durch umlaufende Ventilflächen der Austragvorrichtung gebildet wird, so dass beim Öffnen ein umlaufender Spalt gebildet ist, durch den Luft unbehindert einströmen kann. Hierdurch wird ein besonders geringer Strömungswiderstand am Belüftungsventil erzielt werden, so dass selbst bei nur kurzzeitigem Öffnen des Belüftungsventils dieses dem Druckausgleich nicht im Wege steht.

Ein erfindungsgemäßer Spender weist eine Basis auf der Seite des Flüssigkeitsspeichers und einen gegenüber der Basis zum Zwecke der Betätigung der Pumpeinrichtung verlagerbaren Austragkopf auf. Dabei ist am Austragkopf vorzugsweise auch die Austragöffnung vorgesehen.

Die Basis und der Austragkopf verfügen über ein erstes Gehäuseteil und ein zweites Gehäuseteil aus einem starren Kunststoff, wobei das erste Gehäuseteil an der Basis und das zweite Gehäuseteil am Austragkopf vorgesehen ist. Im Falle der Basis handelt es sich bei dem starren Gehäuseteil vorzugsweise um eines, welches gleichzeitig auch eine Kopplungseinrichtung zur Ankopplung an den Flüssigkeitsspeicher bildet, beispielsweise in Form einer Schnappeinrichtung oder eines Gewindes.

Unter solchen starren Gehäuseteilen werden im Sinne der Erfindung Gehäuseteile verstanden, die auseinem Kunststoff mit einem E-Modul von mehrals 100 N/mm² hergestellt sind, vorzugsweise von mindestens 200 N/mm², insbesondere vorzugsweise von mindestens 800 N/mm². Ein solches starres Material wie beispielsweise PP oder LDPE ist für die meisten Anforderungen des Austragkopfes passend und auch üblich. FürVentilflächen eines Belüftungsventils, welches den einzigen Auslaufschutz bildet, ist es jedoch meist zu starr, denn schon geringe Toleranzen in den Bauteilen können dafür verantwortlich sein, dass es nicht mehr zu einer flächigen Anlage der Ventilflächen kommt.

Das Belüftungsventil wird durch eine erste Ventilfläche und eine zweite Ventilfläche gebildet, die am ersten und am zweiten Gehäuseteil vorgesehen sind, wobei die Ventilflächen bei unbetätigter Pumpeinrichtung aneinander anliegen. Insbesondere vorzugsweise bilden das erste Gehäuseteil und das zweite Gehäuseteil gemeinsam einen Anschlag, der ein Abziehen des Austragkopfes vor der Basis verhindert. Hierbei können insbesondere die Ventilflächen selbstAnschlagflächen des Anschlags bilden.

Die Anschlagflächen und damit vorzugsweise auch die damit identischen Ventilflächen werden üblicherweise von der bereits genannten Rückstellfeder aneinandergedrückt.

Der Kapillarkanalabschnitt ist bei einer erfindungsgemäßen Gestaltung üblicherweise bezogen auf eine Einströmrichtung der Luft stromabwärts des Belüftungsventils angeordnet. Zwar ist grundsätzlich auch eine umgekehrte Reihenfolge möglich. Insbesondere um bei einer nur kurzen Betätigung in einem im Weiteren noch beschriebenen Zwischenraum Umgebungsdruck herzustellen, ist es jedoch von Vorteil, wenn der Belüftungskanal stromaufwärts mit dem Belüftungsventil beginnt.

Bevorzugt ist es, wenn die Pumpeinrichtung eines erfindungsgemäßen Flüssigkeitsspenders über eine zylindrische Pumpkammerwandung sowie über einen innerhalb der Pumpkammerwandung verlagerbaren Pumpenkolben verfügt. Das Belüftungsventil kann bei einer solchen Gestaltung innerhalb der Pumpkammerwandung angeordnet sein, insbesondere wenn der Belüftungskanal abschnittsweise durch den Pumpenkolben verläuft.

Bevorzugt ist es allerdings, wenn das Belüftungsventil außenseitig der Pumpkammerwandung angeordnet ist, insbesondere im Bereich des oben bereits genannten Anschlages. Ein von der Pumpeinrichtung derart getrenntes Belüftungsventil ist konstruktiv einfacher zu gestalten. Es kann insbesondere durch Ventilflächen gebildet sein, von denen mindestens eine Ventilfläche am distalen Ende eines ringförmigen Stegs vorgesehen ist, der die Pumpkammer konzentrisch umgibt.

Der Kapillarkanalabschnitt ist vorzugsweise zwischen zwei Hülsenbauteilen angeordnet, die kraftschlüssig oder formschlüssig miteinander verbunden sind. Die beiden Hülsenbauteile bilden jeweils Wandungen des Kapillarkanalabschnittes.

Die beiden Hülsenbauteile weisen im Bereich des Kapillarkanalabschnitts vorzugsweise zylindrische Teilabschnitte (Öffnungswinkel <1°) auf, die einseitig oder beidseitig mit Vertiefungen versehen sind, die den Kapillarkanalabschnitt bilden. Auch ist es möglich, dass der Kapillarkanalabschnitt im Bereich konischer Teilabschnitte (Öffnungswinkel ≥1°) der Hülsenbauteile vorgesehen ist. Dies kann die Einbringung der mindestens einseitigen Vertiefungen bei der Herstellung der Hülsenbauteile als Kunststoffspritzgussteile vereinfachen, beispielsweise durch die Einbringung einer Art Stufung an einem Hülsenbauteil, welches durch das zweite Hülsenbauteil einen dreieckigen Querschnitt des Kapillarkanalabschnitts verursacht.

Insbesondere vorzugsweise weist der Kapillarkanalabschnitt eine helixförmige Form auf, indem er das innere Hülsenbauteil schraubenförmig umgibt. Im Falle des Vorsehens des Kapillarkanalabschnitts im Bereich konischer Formgebung der Hülsenbauteile ergibt sich eine Helixform mit sich verringerndem Durchmesser.

Zusätzlich zu dem erfindungsgemäß vorgesehenen Belüftungsventil sowie dem erfindungsgemäß vorgesehenen Kapillarkanal ist der Belüftungskanal vorzugsweise um einen Filter als dritte Komponente versehen, die ebenfalls einem Auslaufen des Flüssigkeitsspenders entgegenwirkt. Dieser Filter ist vorzugsweise bezogen auf die Einströmrichtung stromabwärts vom Belüftungsventil und dem Kapillarkanalabschnitt vorgesehen.

Der Filter, bei dem es sich insbesondere um einen Membranfilter handeln kann, verhindert durch diese Anordnung bereits recht zuverlässig das Einströmen von Flüssigkeit in den Kapillarkanal. Die Porengröße des Filters kann so bemessen sein, dass Flüssigkeit üblicherweise nur bei sehr geringem Umgebungsdruck durch den Filter hindurchtreten kann.

Um zu verhindern, dass Flüssigkeit dauerhaft am Filter anliegt oder dort verbleibt, wenn der Spender aus einer liegenden Lage wieder in die aufrechte Lage gebracht wird, ist es von Vorteil, wenn der Filter zumindest an der in Richtung des Flüssigkeitsspeichers gerichteten Seite hydrophob ausgebildet ist. Untereinersolchen Hydrophobie wird ein Kontaktwinkel mit Wasser von mindestens 90° verstanden, vorzugsweise von mindestens 110°.

Das Belüftungsventil und der Kapillarkanalabschnitt wirken beim Einströmen von Luft in den Flüssigkeitsspeicher derart zusammen, dass der durch den Kapillarkanalabschnitt wirkende Unterdruck des Flüssigkeitsspeichers ein Einströmen von Luft in den Kapillarkanalabschnitt bewirkt, die zuvor am Belüftungsventil vorbeigeströmt ist. Aufgrund des hohen Strömungswiderstandes erfolgt der durch den Kapillarkanalabschnitt bewirkte Druckausgleich jedoch langsam.

Um zu ermöglichen, dass trotz der üblicherweise nur kurzzeitigen Öffnung des Belüftungsventil ein ausreichender Druckausgleich erfolgen kann, ist es bevorzugt, wenn im Belüftungskanal eine Zwischenkammer vorgesehen ist, die als Pufferkammer agiert und insbesondere zwischen dem Kapillarkanalabschnitt und dem Belüftungsventil angeordnet ist.

Diese Zwischenkammer bewirkt, dass auch nach dem Schließen des Belüftungsventils noch ein Druckausgleich im Flüssigkeitsspeicher erfolgt, nämlich durch eine Angleichung des Drucks zwischen Flüssigkeitsspeicher und Zwischenkammer. Wird das Belüftungsventil geöffnet, so erfolgt ein Druckausgleich in der Zwischenkammer üblicherweise sofort, da der Strömungswiderstand zwischen der umgebenden Atmosphäre und der Zwischenkammer bei geöffnetem Belüftungsventil sehr gering ist. Nach dem Schließen des Belüftungsventils besteht in der Zwischenkammer zu nächst Umgebungsdruck, bis durch den Kapillarkanalabschnitt eine Druckangleichung zum Flüssigkeitsspeicher eintritt.

Insbesondere vorzugsweise weist die Zwischenkammer ein Volumen auf, welches auf das Pumpenhubvolumen der Pumpeinrichtung abgestimmt ist. Unter dem Pumpenhubvolumen wird das Flüssigkeitsvolumen verstanden, welches mittels der Pumpeinrichtung maximal bei einer Betätigung ausgetragen werden kann. Das Volumen der Zwischenkammer beträgt vorzugsweise mindestens dem 0,5-fachen dieses Pumpenhubvolumens. Um unter der Annahme, dass der Druckausgleich des Flüssigkeitsspeichers ausschließlich nach dem Schließen des Belüftungsventils erfolgt, dennoch einen zumindest weitgehenden Druckausgleich zu erzielen, beträgt das Volumen der Zwischenkammer, worunter insbesondere das Gesamtvolumen zwischen Belüftungsventil und Kapillarkanalabschnitt verstanden wird, vorzugsweise mindestens dem Pumpenvolumen. Um auch im Falle eines Austrags mit mehreren Pumpenhüben einen ausreichenden Ausgleich zu schaffen, wird es bevorzugt, wenn das Volumen der Zwischenkammer mindestens dem 2,0-fachen, insbesondere vorzugsweise mindestens dem 5,0-fachen, des Pumpenhubvolumens der Pumpeinrichtung entspricht.

Das Pumpenhubvolumen der Pumpeinrichtung beträgt vorzugsweise zwischen 0,01 ml und 0,25 ml, insbesondere zwischen 0,05 ml und 0,15 ml Das Volumen der Zwischenkammer beträgt vorzugsweise mindestens 0,005 ml, insbesondere vorzugsweise mindestens 0,02 ml.

Ein erfindungsgemäßer Flüssigkeitsspender dient vorzugsweise insbesondere der Ausbringung einer kosmetischen Flüssigkeit oder einer pharmazeutischen Flüssigkeit. Der erfindungsgemäße Flüssigkeitsspender ist daher in einem Lieferzustand vorzugsweise mit einer solchen Flüssigkeit befüllt.

Bei pharmazeutischen Flüssigkeiten im Flüssigkeitsspeicher kann es sich insbesondere um eine Nasenspray-Flüssigkeit, insbesondere mit dem Inhaltsstoff Metazoline, um eine Flüssigkeit mit dem Wirkstoff Triptane, um eine Saline-Lösung, um eine Flüssigkeit mit einem Schmerzmittel-Wirkstoff, um eine Flüssigkeit mit Antihistamine, um eine Flüssigkeit mit Antiallergiemittel, um eine Augentropfen- oder Augenspray-Flüssigkeit, um eine Flüssigkeit zur Ausbringung als dermales oder orales Spray, um eine Inhalationsflüssigkeit, um eine Desinfektionsspray-Flüssigkeit oder im eine Flüssigkeit zur Waschung von Wunden handeln.

Bei kosmetischen Flüssigkeiten im Flüssigkeitsspeicher kann es sich insbesondere um Seife oder Lotionen handeln. Weiterhin zählten zu den kosmetischen Flüssigkeiten Perfumes, Hautreinigungsfluide und Makeup-Entferner.

### KURZBESCHREIBUNG DER ZEICHNUNGEN

Weitere Vorteile und Aspekte der Erfindung ergeben sich aus den Ansprüchen und aus der nachfolgenden Beschreibung von bevorzugten Ausführungsbeispielen der Erfindung, die nachfolgend anhand der Figuren erläutert sind.
Fig. 1A zeigt einen erfindungsgemäßen Nasalspender in einem Ruhezustand mit geschlossenem Belüftungskanal.
Fig. 1B zeigt den Nasalspender im betätigten Zustand mit geöffnetem Belüftungskanal.
Fig. 2 zeigt eine alternative Ausgestaltung des Nasalspenders der Fig. 1A und 1B mit einem verbesserten Belüftungsventil.
Fig. 3 zeigt eine weitere alternative Gestaltung mit einem anders geführten Belüftungskanal.
Fig. 4 zeigt den Austragkopf eines Sprühspenders mit lateral ausgerichteter Austragöffnung.
Fig. 5A und 5B zeigen ein Hülsenbauteil zur Bildung eines Kapillarkanalabschnitts des Belüftungskanals.

### DETAILLIERTE BESCHREIBUNG DER AUSFÜHRUNGSBEISPIELE

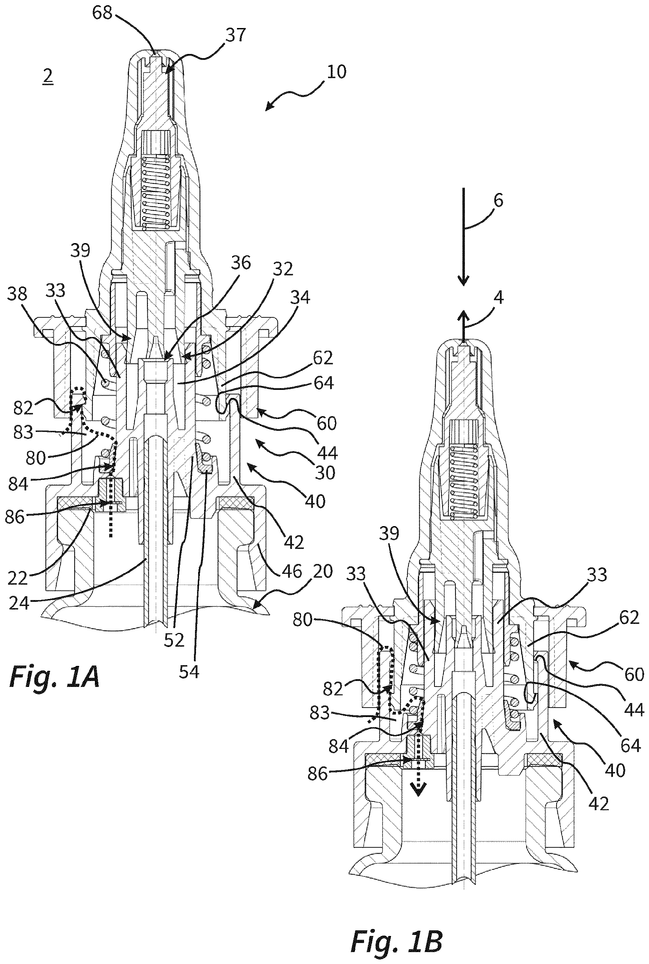

Die Fig. 1A und 1B zeigen einen Flüssigkeitsspender 10 mit einem nur partiell dargestellten Flüssigkeitsspeicher 20, auf den eine Austragvorrichtung 30 aufgesetzt ist. Der Flüssigkeitsspender 10 der Fig. 1A und 1B ist als Nasalspender ausgebildet. Die Austragvorrichtung 30 verfügt über eine Basis 40, die mittels eines Schnapprings 46 am Flüssigkeitsspeicher 20 befestigt ist, wobei durch eine Dichtung 22 verhindert wird, dass Flüssigkeit aus dem Flüssigkeitsspeicher an der Austragvorrichtung 30 vorbei entweichen kann.

Neben der Basis 40 verfügt die Austragvorrichtung 30 über einen in Richtung des Pfeils 6 der Fig. 1B niederdrückbaren Austragkopf 60, an dessen distalen Ende die Austragöffnung 68 vorgesehen ist. Gemeinsam bilden die Basis 40 und der demgegenüber bewegliche Austragkopf 60 eine Pumpeinrichtung 32. Diese Pumpeinrichtung verfügt auf der Seite der Basis über eine Pumpkammerwandung 33, innerhalb derer ein zum Austragkopf 60 gehörender Pumpenkolben 39 beweglich ist, so dass durch diese Bewegung das Volumen der Pumpkammer 34 gegen die Kraft einer Rückstellfeder 38 reduzierbar ist. Die Pumpeinrichtung 32 verfügt über zwei Ventile, nämlich ein Einlassventil 36 und ein federvorgespanntes Auslassventil 37. Wenn bei befüllter Pumpkammer 34 der Austragkopf 60 in der in Fig. 1B verdeutlichten Weise niedergedrückt wird, so wird hierdurch das Einlassventil 36 geschlossen, so dass eine fortgesetzte Bewegung des Austragkopfes 60 in Richtung des Pfeils 6 nach unten zu einer Druckerhöhung der Flüssigkeit in der Pumpkammer 34 führt, welche wiederrum ein Öffnen des Auslassventils und einen Austrag der Flüssigkeit in Richtung des Pfeils 4 bewirkt. Beim Rückhub wird weitere Flüssigkeit durch ein Steigrohr24 in die Pumpkammer34 eingesogen.

Im Zuge eines Flüssigkeitsaustrages, insbesondere eines Flüssigkeitsaustrages mit mehreren aufeinander unmittelbar folgenden Pumpenhüben, entsteht im Flüssigkeitsspeicher 20 ein Unterdruck. Um diesen Unterdruck wieder abzubauen, ist ein Belüftungskanal 80 vorgesehen, der in den Fig. 1A und 1B durch eine gepunktete Linie dargestellt ist und der im Zustand der Fig. 1A geschlossen und im Zustand der Fig. 1B geöffnet ist.

Bezugnehmend auf Fig. 1A verläuft dieser Belüftungskanal durch einen Ringspalt an der Innenseite einer Fingerauflage des Austragkopfes 60 bis zu einem Belüftungsventil 82, welches durch Anschlagflächen 44, 64 der Basis 40 und des Austragkopfes 60 gebildet ist. Hieran schließt sich eine ringförmige Zwischenkammer 83 an, die vorliegend etwa das fünffache Volumen eines Pumpenhubs der Pumpeinrichtung 32 aufweist. Weiter stromabwärts folgt im Belüftungskanal 80 ein Kapillarkanalabschnitt, der zwischen zwei Hülsenbauteilen 52, 54 gebildet ist. Das Hülsenbauteil 52 ist dabei identisch mit einem Hauptbauteil der Basis 40. Das Hülsenbauteil 54 wird durch eine separate Hülse gebildet, welche auf das Hülsenbauteil 52 aufgeschoben ist und kraftschlüssig hier in Position verbleibt. In einer der Fig. 1A und 1B nicht klar ersichtlichen Weise ist an der Innenseite des Hülsenbauteils 54 eine helixförmige Nut eingebracht, die nach dem kraftschlüssigen Aufdrücken auf das innere Hülsenbauteil 52 den helixförmigen Kapillarkanalabschnitt bildet. Dieser Kapillarkanalabschnitt hat eine Länge von etwa 40 mm und eine lichte Weite von etwa 0,02 mm.

Weiter stromabwärts im Belüftungskanal 80 schließt sich als letztes Element vor dem Flüssigkeitsspeicher ein Filter 86 an, dessen den Flüssigkeitsspeicher 20 zugewandte Seite hydrophob ausgestaltet ist.

Im Ruhezustand des Flüssigkeitsspenders 10 ist das Belüftungsventil 82 in der beschriebenen Weise geschlossen. Wenn nun der Flüssigkeitsspender betätigt wird und Flüssigkeit in der eingangs beschriebenen Weise ausgetragen wird, stellt sich während des Rückhubs im Flüssigkeitsspeicher 20 ein Unterdruck ein, der zunächst nicht ausgeglichen wird. Erst in den Minuten nach der Verwendung des Spenders und bei bereits wieder geschlossenem Belüftungsventil 82 erfolgtein Ausgleich, nämlich dadurch, dass Luft aus der Zwischenkammer 83 durch den Kapillarkanalabschnitt 84 und den Filter 86 in den Flüssigkeitsspeicher 20 gelangen. Dies führt zwar nichtzu einem vollständigen Druckausgleich im Flüssigkeitsspeicher 20, jedoch zu einem weitgehenden. Nach längerer Ruhezeit verbleibt somit ein in etwa einheitlicher leichter Unterdruck sowohl im Flüssigkeitsspeicher 20 als auch in der Zwischenkammer 83. Wird nun der Spender ein nächstes Mal betätigt, so erhöht sich während des Rückhubs wieder der Unterdruck im Flüssigkeitsspeicher 20. Gleichzeitig jedoch kommt es durch das entsprechend der Fig. 1B geöffnete Belüftungsventil 82 unmittelbar zu einem vollständigen Druckausgleich in der Zwischenkammer 83. Nachdem das Belüftungsventil 82 wieder geschlossen ist, kann somit in der bereits beschriebenen Art und Weise ein weitgehender Druckausgleich im Flüssigkeitsspeicher durch Einströmen von Luft aus der Zwischenkammer 83 stattfinden.

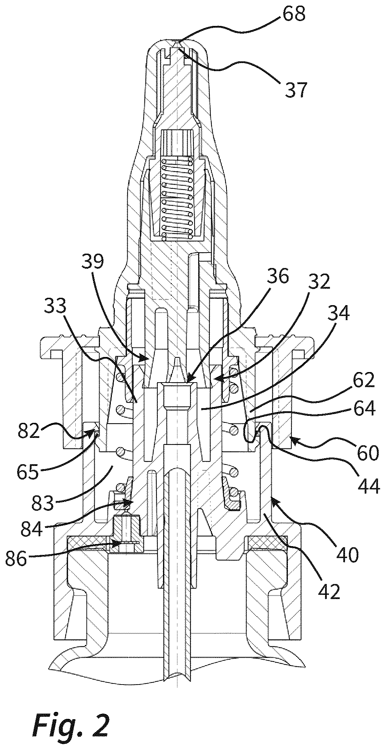

Fig. 2 zeigt eine Variante zur Gestaltung der Fig. 1A und 1B. Einziger Unterschied ist, dass bei dieser Gestaltung am Belüftungsventil ein Dichtungsring 65 aus einem elastisch verformbaren Material wie insbesondere einem gummiartigen Material vorgesehen ist. Dieser Dichtungsring 65 kann entweder fest an der Basis 40 oder fest am Austragkopf 60 vorgesehen sein. Der Dichtungsring verbessert die Dichtwirkung des Belüftungsventils 82, so dass in höherem Maße gewährleistet ist, dass das Belüftungsventil 82 auch nach längerer Lager- oder Liegezeit vollständig dicht abschließt. Wenngleich eine solche Gestaltung technisch vorteilhaft ist, so ist sie meist wirtschaftlich von Nachteil. Der zusätzliche Dichtring verursacht Kosten, die bei der erfindungsgemäßen Gestaltung an sich nicht erforderlich sind. Durch die hintereinander in Serie angeordneten Elemente des Belüftungsventils und des Kapillarkanals sowie des zusätzlichen Filters ist ein Auslaufschutz auch dann gewährleistet, wenn das Belüftungsventil 82 nicht vollständig schließt.

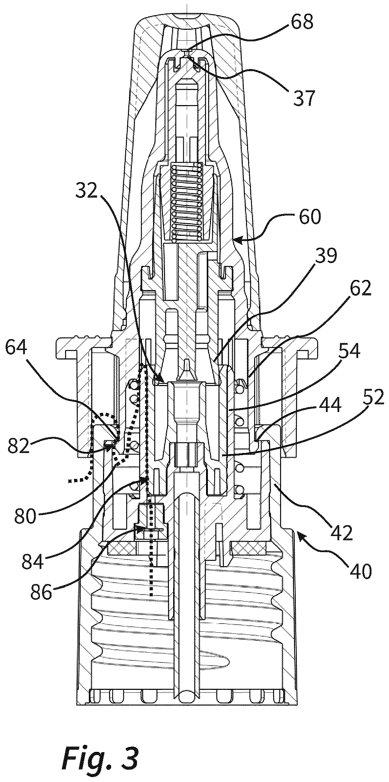

Fig. 3 zeigt eine weitere Variante eines erfindungsgemäßen Flüssigkeitsspenders, wobei vorliegend zu Vereinfachungszwecken nur die Austragvorrichtung 30 des Flüssigkeitsspenders dargestellt ist. Der wesentliche Unterschied zur Gestaltung der Fig. 1A und 1B liegt darin, dass der Kapillarkanalabschnitt 84 hier anders realisiert ist. Wie der Figurzu entnehmen ist, ist der Kapillarkanalabschnitt 84 durch zwei Hülsenbauteile 52, 54 gebildet, wobei das innere Hülsenbauteil 52 als separates Bauteil ausgebildet ist und mit seiner Innenseite eine Lauffläche für den Pumpenkolben 39 zur Verfügung stellt. An der Außenseite dieses inneren Hülsenbauteils 52 ist wiederrum eine helixförmige Nut eingebracht, die den Kapillarkanalabschnitt bildet.

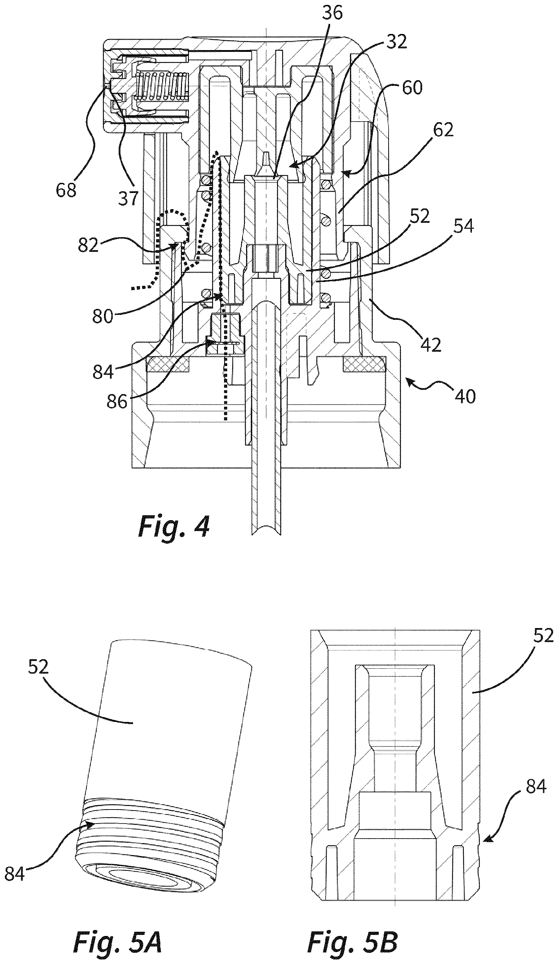

Die Gestaltung der Fig. 4 ist in Hinblick auf die erfindungswesentlichen Bauteile nahezu identisch zur Gestaltung der Fig. 3. Hier ist es jedoch kein Nasalspender, sondern ein Spender zum lateralen Austrag von Flüssigkeit, der in der erfindungsgemäßen Art und Weise mit einem Belüftungskanal 80 ausgebildet ist.

Die Fig. 5A und 5B zeigen das Hülsenbauteil 52 des Flüssigkeitsspenders gemäß Fig. 3, wobei die Unterschiede zum entsprechenden Hülsenbauteil 54 der Gestaltung der Fig. 4 sich in erfindungsunwesentlichen Details erschöpfen. Es ist zu erkennen, dass das Hülsenbauteil 52 an seinem unteren Ende mit einer helixförmigen Vertiefung vorgesehen ist, die im Zusammenwirkung mit der Innenseite des Hülsenbauteils 52 zu einem helixförmigen Belüftungskanal führt.

## Patentansprüche

1. Flüssigkeitsspender (10) mit den folgenden Merkmalen:
a. der Flüssigkeitsspender (10) verfügt über einen Flüssigkeitsspeicher (20) zur Lagerung von Flüssigkeit vor dem Austrag, und
b. der Flüssigkeitsspender (10) verfügt über eine am Flüssigkeitsspeicher (20) angebrachte Austragvorrichtung (30) mit einer Austragöffnung (68) zum Austrag der Flüssigkeit, und
c. die Austragvorrichtung (30) verfügt über eine Pumpeinrichtung (32) zur manuellen Betätigung mit einer volumetrisch veränderlichen Pumpkammer (34), die an einer Eingangsseite kommunizierend mit dem Flüssigkeitsspeicher (20) und an einer Ausgangsseite kommunizierend mit der Austragöffnung (68) verbunden ist, und
d. die Austragvorrichtung (30) verfügt über einen Belüftungskanal (80), mittels dessen ein einfließender Luftstrom zum Druckausgleich zwischen dem Flüssigkeitsspeicher (20) und einer umgebende Atmosphäre (2) ermöglicht wird, und
e. der Belüftungskanal (80) verfügt über ein Belüftungsventil (82), welches durch manuelle Betätigung der Pumpeinrichtung (32) geöffnet werden kann und welches bei unbetätigter Pumpeinrichtung (32) geschlossen ist, und
f. der Belüftungskanal (80) verfügt übereinen Kapillarkanalabschnitt (84), durch den dereinfließende Luftstrom in den Flüssigkeitsspeicher gelangt,
**gekennzeichnet durch** die folgenden zusätzlichen Merkmale:
g. die Austragvorrichtung (30) weist eine Basis (40) auf der Seite des Flüssigkeitsspeichers (20) und einen gegenüber der Basis (40) zum Zwecke der Betätigung der Pumpeinrichtung verlagerbaren Austragkopf (60) auf, und
h. die Basis (40) und der Austragkopf (60) verfügen über ein erstes Gehäuseteil (42) und ein zweites Gehäuseteil (62) aus einem starren Kunststoff, wobei das erste Gehäuseteil (42) an der Basis (40) und das zweite Gehäuseteil (62) am Austragkopf (60) vorgesehen ist, und
i. das Belüftungsventil (82) ist durch eine erste Ventilfläche (44) und eine zweite Ventilfläche gebildet (64), die am ersten und am zweiten Gehäuseteil (42, 62) vorgesehen sind, wobei die Ventilflächen (44, 64) bei unbetätigter Pumpeinrichtung (32) aneinander anliegen.

2. Flüssigkeitsspender (10) nach Anspruch 1 mit dem folgenden weiteren Merkmal:
a. das erste Gehäuseteil (42) und das zweite Gehäuseteil (62) bilden gemeinsam einen Anschlag, der ein Abziehen des Austragkopfes (60) vor der Basis (40) verhindert,
insbesondere mit dem zusätzlichen Merkmal:
b. die beiden Ventilflächen (44, 64) bilden Anschlagsflächen des Anschlags.

3. Flüssigkeitsspender (10) nach einem der vorstehenden Ansprüche mit dem folgenden weiteren Merkmal:
a. der Kapillarkanalabschnitt (84) ist bezogen auf eine Einströmrichtung der Luft stromabwärts des Belüftungsventils (82) angeordnet.

4. Flüssigkeitsspender nach einem der vorstehenden Ansprüche mit den folgenden weiteren Merkmalen:
a. die Pumpeinrichtung (32) verfügt über eine zylindrische Pumpkammerwandung (33) sowie über einen innerhalb der Pumpkammerwandung (33) verlagerbaren Pumpenkolben (39), und
b. das Belüftungsventil (82) ist außenseitig der Pumpkammerwandung (33) angeordnet.

5. Flüssigkeitsspender (10) nach einem der vorstehenden Ansprüche mit mindestens einem der folgenden weiteren Merkmale:
a. der Kapillarkanalabschnitt (84) weist eine Länge größer als 10 mm auf, vorzugsweise größer als 30 mm ist, insbesondere größer als 50 mm, und/oder
b. eine mittlere Querschnittsfläche des Kapillarkanals ist kleiner als 0,05 mm², vorzugsweise kleiner als 0,02 mm², insbesondere vorzugsweise kleiner als 0,01 mm², und/oder
c. der Kapillarkanalabschnitt (84) weist einen minimalen lichten Querschnitt von weniger als 0,02 mm² auf, und/oder
d. der Quotient aus der Länge des Kapillarkanalabschnitts (84) geteilt durch seine mittlere Querschnittsfläche ist größer als 300 mm⁻¹, insbesondere größer als 1000 mm⁻¹.

6. Flüssigkeitsspender (10) nach einem der vorstehenden Ansprüche mit dem folgenden weiteren Merkmal:
a. der Kapillarkanalabschnitt (84) ist zwischen zwei Hülsenbauteilen (52, 54) angeordnet, die kraftschlüssig oder formschlüssig miteinander verbunden sind,
insbesondere mit einem der folgenden zusätzlichen Merkmale:
b. der Kapillarkanalabschnitt (84) ist zumindest teilweise an einem zylindrischen Teilabschnitt der Hülsenbauteile vorgesehen, und/oder
c. der Kapillarkanalabschnitt (84) ist zumindest teilweise an einem konischen Teilabschnitt der Hülsenbauteile vorgesehen, und/oder
d. der Kapillarkanalabschnitt (84) ist durch eine helixförmige Vertiefung an einem der Hülsenbauteile gebildet, insbesondere einer Vertiefung mit dreieckigem Querschnitt.

7. Flüssigkeitsspender (10) nach einem der vorstehenden Ansprüche mit dem folgenden zusätzlichen Merkmal:
a. der Belüftungskanal (80) weist einen Filter (86) auf, insbesondere einen Membranfilter,
insbesondere mit dem folgenden Merkmal:
b. der Filter (86) ist bezogen auf eine Einströmrichtung der Luft stromabwärts des Belüftungsventils (82) und/oder stromabwärts des Kapillarkanalabschnitts (84) angeordnet.

8. Flüssigkeitsspender (10) nach Anspruch 7 mit dem folgenden weiteren Merkmal:
a. der Filter (86) ist zumindestan einer in Richtung des Flüssigkeitsspeichers gerichteten Seite hydrophob ausgebildet.

9. Flüssigkeitsspender nach einem der vorstehenden Ansprüche mit den folgenden weiteren Merkmalen:
a. der Belüftungskanal (80) weist eine Zwischenkammer (83) auf, die insbesondere zwischen dem Kapillarkanalabschnitt (84) und dem Belüftungsventil (82) angeordnet ist, und
b. die Zwischenkammer weist ein Volumen auf, welches mindestens dem 0,5-fachen eines Pumpenhubvolumens der Pumpeinrichtung (32) entspricht,
insbesondere mit dem folgenden zusätzlichen Merkmal:
c. die Zwischenkammer weist ein Volumen auf, welches mindestens dem 1,0-fachen eines Pumpenhubvolumens der Pumpeinrichtung (32) entspricht, vorzugsweise mindestens dem 2,0-fachen, insbesondere vorzugsweise mindestens dem 5,0-fachen des Pumpenhubvolumens der Pumpeinrichtung (32).

10. Flüssigkeitsspender nach einem der vorstehenden Ansprüche mit dem folgenden weiteren Merkmal:
a. ein Pumpenhubvolumen der Pumpeinrichtung (32) beträgt zwischen 0,01 ml und 0,25 ml, insbesondere zwischen 0,05 ml und 0,15 ml.

11. Flüssigkeitsspender (10) nach einem der vorstehenden Ansprüche mit mindestens einem der folgenden weiteren Merkmale:
a. die Ventilflächen des Belüftungsventils liegen mit einem mittleren Anpressdruck von 0,5 N/mm² bis 1,5 N/mm² aneinander an und/oder
b. es ist eine der Pumpeinrichtung zugeordnete Rückstellfeder vorgesehen, die die Ventilflächen des Belüftungsventils aneinander anpresst.

12. Flüssigkeitsspender (10) nach einem der vorstehenden Ansprüche mit einem der folgenden weiteren Merkmale:
a. der Flüssigkeitsspeicher (20) ist mit einer pharmazeutischen Flüssigkeit befüllt, insbesondere mit einer
- Nasenspray-Flüssigkeit, insbesondere mit dem Inhaltsstoff Metazoline, oder
- Flüssigkeit mit dem Wirkstoff Triptane, oder
- Saline-Lösung, oder
- Flüssigkeit mit einem Schmerzmittel-Wirkstoff, oder
- Flüssigkeit mit Antihistamine, oder
- Flüssigkeit mit Antiallergiemittel, oder
- Augentropfen- oder Augenspray-Flüssigkeit, oder
- Flüssigkeit zur Ausbringung als dermales oder orales Spray, oder
- Inhalationsflüssigkeit, oder
- Desinfektionsspray-Flüssigkeit, oder
- Flüssigkeit zur Waschung von Wunden, oder
b. der Flüssigkeitsspeicher (20) ist mit einer kosmetischen Flüssigkeit befüllt, insbesondere mit
- einem Parfumes, oder
- einem Hautreinigungsfluid, oder
- einem Makeup-Entferner.

## Claims

1. Liquid dispenser (10) having the following features:
a. the liquid dispenser (10) has a liquid store (20) for storing liquid prior to the discharge, and
b. the liquid dispenser (10) has a discharge device (30) which is fitted to the liquid store (20) and which has a discharge opening (68) for discharging the liquid, and
c. the discharge device (30) has a pump device (32) for manual activation with a pump chamber (34), which is variable in terms of volume and which is connected at an input side in a communicating manner to the liquid store (20) and which is connected at an output side in a communicating manner to the discharge opening (68), and
d. the discharge device (30) has a ventilation channel (80), by means of which an incoming air flow is enabled for pressure compensation between the liquid store (20) and a surrounding atmosphere (2), and
e. the ventilation channel (80) has a ventilation valve (82) which can be opened by means of manual activation of the pump device (32) and which is closed when the pump device (32) is inactive, and
f. the ventilation channel (80) has a capillary channel portion (84), through which the incoming air flow reaches the liquid store,
**characterized by** the following additional features:
g. the discharge device (30) has a base (40) at the side of the liquid store (20) and a discharge head (60) which can be displaced relative to the base (40) for the purposes of activating the pump device, and
h. the base (40) and the discharge head (60) have a first housing portion (42) and a second housing portion (62) of a rigid plastics material, wherein the first housing portion (42) is provided on the base (40) and the second housing portion (62) is provided on the discharge head (60), and
i. the ventilation valve (82) is formed by means of a first valve face (44) and a second valve face (64) which are provided on the first and second housing portions (42, 62), wherein the valve faces (44, 64) are in abutment with each other when the pump device (32) is not activated.

2. Liquid dispenser (10) according to Claim 1 having the following further feature:
a. the first housing portion (42) and the second housing portion (62) together form a stop which prevents the discharge head (60) from being removed from the base (40),
in particular having the additional feature:
b. the two valve faces (44, 64) form stop faces of the stop.

3. Liquid dispenser (10) according to any one of the preceding claims having the following further feature:
a. the capillary channel portion (84) is arranged with respect to an influx direction of the air downstream of the ventilation valve (82).

4. Liquid dispenser according to any one of the preceding claims having the following further features:
a. the pump device (32) has a cylindrical pump chamber wall (33) and a pump piston (39) which can be displaced inside the pump chamber wall (33) and
b. the ventilation valve (82) is arranged at the outer side of the pump chamber wall (33).

5. Liquid dispenser (10) according to any one of the preceding claims having at least one of the following further features:
a. the capillary channel portion (84) has a length of greater than 10 mm, preferably greater than 30 mm, in particular greater than 50 mm, and/or
b. a mean cross-sectional surface-area of the capillary channel is less than 0.05 mm², preferably less than 0.02 mm², particularly preferably less than 0.01 mm², and/or
c. the capillary channel portion (84) has a minimum clear cross-section of less than 0.02 mm², and/or
d. the quotient of the length of the capillary channel portion (84) divided by the mean cross-sectional surface-area thereof is greater than 300 mm⁻¹, in particular greater than 1000 mm⁻¹.

6. Liquid dispenser (10) according to any one of the preceding claims having the following further feature:
a. the capillary channel portion (84) is arranged between two sleeve components (52, 54) which are connected to each other in a non-positive-locking or positive-locking manner,
in particular having one of the following additional features:
b. the capillary channel portion (84) is at least partially provided on a cylindrical part-portion of the sleeve components, and/or
c. the capillary channel portion (84) is at least partially provided on a conical part-portion of the sleeve components, and/or
d. the capillary channel portion (84) is formed by means of a helical recess in one of the sleeve components, in particular a recess having a triangular cross-section.

7. Liquid dispenser (10) according to any one of the preceding claims having the following additional feature:
a. the ventilation channel (80) has a filter (86), in particular a membrane filter,
in particular having the following feature:
b. the filter (86) is with respect to an influx direction of the air arranged downstream of the ventilation valve (82) and/or downstream of the capillary channel portion (84).

8. Liquid dispenser (10) according to Claim 7 having the following further feature:
a. the filter (86) is constructed at least at a side facing in the direction of the liquid store in a hydrophobic manner.

9. Liquid dispenser according to any one of the preceding claims having the following further features:
a. the ventilation channel (80) has an intermediate chamber (83) which is arranged in particular between the capillary channel portion (84) and the ventilation valve (82), and
b. the intermediate chamber has a volume which corresponds to at least 0.5 times a pump stroke volume of the pump device (32),
in particular having the following additional feature:
c. the intermediate chamber has a volume which corresponds to at least 1.0 times a pump stroke volume of the pump device (32), preferably at least 2.0 times, in a particularly preferred manner at least 5.0 times, the pump stroke volume of the pump device (32).

10. Liquid dispenser according to any one of the preceding claims having the following further feature:
a. a pump stroke volume of the pump device (32) is between 0.01 ml and 0.25 ml, in particular between 0.05 ml and 0.15 ml.

11. Liquid dispenser (10) according to any one of the preceding claims having at least one of the following further features:
a. the valve faces of the ventilation valve are in abutment with each other with a mean pressing pressure of from 0.5 N/mm² to 1.5 N/mm² and/or
b. there is provided a return spring which is associated with the pump device and which presses the valve faces of the ventilation valve against each other.

12. Liquid dispenser (10) according to any one of the preceding claims having one of the following further features:
a. the liquid store (20) is filled with a pharmaceutical liquid, in particular with a
- nasal spray liquid, in particular with the active ingredient metazoline, or
- liquid with the active ingredient triptane, or
- saline solution, or
- liquid with a pain-relieving active ingredient, or
- liquid with antihistamine, or
- liquid with an antiallergenic agent, or
- eye drop or eye spray liquid, or
- liquid for discharge as a dermal or oral spray, or
- inhalation liquid, or
- disinfecting spray liquid, or
- liquid for washing wounds, or
b. the liquid store (20) is filled with a cosmetic liquid, in particular with
- a perfume, or
- a skin cleansing fluid, or
- a makeup remover.

## Revendications

1. Distributeur de liquide (10) présentant les caractéristiques suivantes :
a. le distributeur de liquide (10) dispose d'un réservoir de liquide (20) pour le stockage de liquide avant le déchargement, et
b. le distributeur de liquide (10) dispose d'un dispositif de déchargement (30) disposé sur le réservoir de liquide (20), avec une ouverture de déchargement (68) pour le déchargement du liquide, et
c. le dispositif de déchargement (30) dispose d'un appareil de pompage (32) pour l'actionnement manuel avec une chambre de pompage (34) variable en volume, qui est reliée à un côté d'entrée en communication avec le réservoir de liquide (20) et à un côté de sortie en communication avec l'ouverture de déchargement (68), et
d. le dispositif de déchargement (30) dispose d'un canal d'aération (80), au moyen duquel un courant d'air entrant est permis pour l'équilibrage de pression entre le réservoir de liquide (20) et une atmosphère environnante (2), et
e. le canal d'aération (80) dispose d'une soupape d'aération (82), qui peut être ouverte par actionnement manuel de l'appareil de pompage (32) et qui est fermée lorsque l'appareil de pompage (32) n'est pas actionné, et
f. le canal d'aération (80) dispose d'une section de canal capillaire (84), à travers laquelle le courant d'air entrant pénètre dans le réservoir de liquide,
**caractérisé par** les caractéristiques supplémentaires suivantes :
g. le dispositif de déchargement (30) présente une base (40) sur le côté du réservoir de liquide (20) et une tête de déchargement (60) déplaçable par rapport à la base (40) dans le but d'actionner l'appareil de pompage, et
h. la base (40) et la tête de déchargement (60) disposent d'une première partie de boîtier (42) et d'une deuxième partie de boîtier (62) en une matière plastique rigide, la première partie de boîtier (42) étant prévue sur la base (40) et la deuxième partie de boîtier (62) sur la tête de déchargement (60), et
i. la soupape d'aération (82) est formée par une première surface de soupape (44) et une deuxième surface de soupape (64), qui sont prévues sur la première et la deuxième partie du boîtier (42, 62), les surfaces de soupape (44, 64) s'appliquant l'une contre l'autre lorsque l'appareil de pompage (32) n'est pas activé.

2. Distributeur de liquide (10) selon la revendication 1, présentant l'autre caractéristique suivante :
a. la première partie de boîtier (42) et la deuxième partie de boîtier (62) forment ensemble une butée, qui empêche un retrait de la tête de déchargement (60) devant la base (40),
en particulier présentant la caractéristique supplémentaire :
b. les deux surfaces de soupape (44, 64) forment des surfaces de butée de la butée.

3. Distributeur de liquide (10) selon l'une quelconque des revendications précédentes, présentant l'autre caractéristique suivante :
a. la section de canal capillaire (84) est agencée en aval de la soupape d'aération (82) par rapport à la direction d'entrée de l'air.

4. Distributeur de liquide selon l'une quelconque des revendications précédentes, présentant les autres caractéristiques suivantes :
a. l'appareil de pompage (32) dispose d'une paroi de chambre de pompage cylindrique (33) ainsi que d'un piston de pompe (39) déplaçable à l'intérieur de la paroi de chambre de pompage (33), et
b. la soupape d'aération (82) est agencée sur le côté extérieur de la paroi de chambre de pompage (33).

5. Distributeur de liquide (10) selon l'une quelconque des revendications précédentes, présentant au moins l'une des autres caractéristiques suivantes :
a. la section de canal capillaire (84) présente une longueur supérieure à 10 mm, de préférence supérieure à 30 mm, en particulier supérieure à 50 mm, et/ou
b. la surface de section transversale moyenne du canal capillaire est inférieure à 0,05 mm², de préférence inférieure à 0,02 mm², en particulier de préférence inférieure à 0,01 mm², et/ou
c. la section de canal capillaire (84) présente une section transversale libre minimale de moins de 0,02 mm², et/ou
d. le quotient de la longueur de la section de canal capillaire (84) divisée par sa surface de section transversale moyenne est supérieur à 300 mm⁻¹, en particulier supérieur à 1 000 mm⁻¹.

6. Distributeur de liquide (10) selon l'une quelconque des revendications précédentes, présentant l'autre caractéristique suivante :
a. la section de canal capillaire (84) est agencée entre deux composants de douille (52, 54), qui sont reliés entre eux par adhérence ou par complémentarité de forme,
en particulier présentant l'une des caractéristiques supplémentaires suivantes :
b. la section de canal capillaire (84) est prévue au moins partiellement sur une section partielle cylindrique des composants de douille, et/ou
c. la section de canal capillaire (84) est prévue au moins partiellement sur une section partielle conique des composants de douille, et/ou
d. la section de canal capillaire (84) est formée par un creux en forme d'hélice sur l'un des composants de douille, en particulier un creux de section transversale triangulaire.

7. Distributeur de liquide (10) selon l'une quelconque des revendications précédentes, présentant la caractéristique supplémentaire suivante :
a. le canal d'aération (80) présente un filtre (86), en particulier un filtre à membrane,
en particulier présentant la caractéristique suivante :
b. le filtre (86) est agencé en aval de la soupape d'aération (82) et/ou en aval de la section de canal capillaire (84) par rapport à la direction d'entrée de l'air.

8. Distributeur de liquide (10) selon la revendication 7, présentant l'autre caractéristique suivante :
a. le filtre (86) est configuré sous forme hydrophobe au moins sur un côté orienté en direction du réservoir de liquide.

9. Distributeur de liquide selon l'une quelconque des revendications précédentes, présentant les autres caractéristiques suivantes :
a. le canal d'aération (80) présente une chambre intermédiaire (83), qui est agencée en particulier entre la section de canal capillaire (84) et la soupape d'aération (82), et
b. la chambre intermédiaire présente un volume qui correspond à au moins 0,5 fois le volume de course de pompage de l'appareil de pompage (32), en particulier présentant la caractéristique supplémentaire suivante :
c. la chambre intermédiaire présente un volume qui correspond à au moins 1,0 fois un volume de course de pompage de l'appareil de pompage (32), de préférence à au moins 2,0 fois, en particulier de préférence à au moins 5,0 fois le volume de course de pompage de l'appareil de pompage (32).

10. Distributeur de liquide selon l'une quelconque des revendications précédentes, présentant l'autre caractéristique suivante :
a. un volume de course de pompage de l'appareil de pompage (32) est compris entre 0,01 ml et 0,25 ml, en particulier entre 0,05 ml et 0,15 ml.

11. Distributeur de liquide (10) selon l'une quelconque des revendications précédentes, présentant au moins l'une des autres caractéristiques suivantes :
a. les surfaces de soupape de la soupape d'aération s'appliquent l'une contre l'autre avec une pression d'application moyenne de 0,5 N/mm² à 1,5 N/mm² et/ou
b. un ressort de rappel associé à l'appareil de pompage est prévu, qui presse les surfaces de la soupape de la soupape d'aération l'une contre l'autre.

12. Distributeur de liquide (10) selon l'une quelconque des revendications précédentes, présentant l'une des autres caractéristiques suivantes :
a. le réservoir de liquide (20) est rempli d'un liquide pharmaceutique, en particulier
- d'un liquide pour pulvérisation nasale, en particulier contenant l'ingrédient métazoline, ou
- d'un liquide contenant l'agent actif triptans, ou
- d'une solution saline, ou
- d'un liquide contenant un agent actif analgésique, ou
- d'un liquide contenant des antihistaminiques, ou
- d'un liquide contenant des agents antiallergiques, ou
- d'un liquide pour collyre ou pulvérisation oculaire, ou
- d'un liquide pour application en tant que pulvérisation cutanée ou orale, ou
- d'un liquide à inhaler, ou
- d'un liquide pour pulvérisation désinfectante, ou
- d'un liquide pour le lavage des plaies, ou
b. le réservoir de liquide (20) est rempli d'un liquide cosmétique, en particulier
- d'un parfum, ou
- d'un fluide de nettoyage de la peau, ou
- d'un démaquillant.
